# EUROPÄISCHE PATENTANMELDUNG

(11) **EP 3 984 565 A1**
(43) Veröffentlichungstag der Anmeldung: **20.04.2022**
(21) Anmeldenummer: 21201881.6
(22) Anmeldetag: 11.10.2021
(51) Int. Cl.: A61L 9/20, F24B 1/188, F24B 7/02

(54) **RAUMLUFTHEIZEINRICHTUNG SOWIE ANORDNUNG EINER RAUMLUFTHEIZEINRICHTUNG**

(30) Priorität: 19.10.2020 DE 102020127478
(71) Anmelder: Schiedel GmbH, 4542 Nussbach (AT)
(72) Erfinder: Elfert, Thomas, 04668 Leipnitz (DE); Paschke, Torsten, 04828 Plagwitz (DE); Pramberger, Christoph, 4553 Schlierbach (AT); Bissinger, Claus, 67271 Kleinkarlbach (DE)
(74) Vertreter: Müller Schupfner & Partner Patent- und Rechtsanwaltspartnerschaft mbB

(57) **Zusammenfassung**

Raumluftheizeinrichtung (1), umfassend eine Luftführungsanordnung (30) und eine Sterilisatoreinheit (50), wobei die Luftführungsanordnung (30) einen Einlass (32) und einen Auslass (34) aufweist, wobei die Luftführungsanordnung (30) dazu ausgelegt ist, einen Fluidstrom (FF), insbesondere einen Luftstrom, von dem Einlass (32) zu dem Auslass (34) zu leiten, wobei die Luftführungsanordnung (30) zumindest eine beheizbare und/oder beheizte Wand (36) aufweist, wobei die Sterilisatoreinheit (50) derart angeordnet ist, dass diese den Fluidstrom (FF) sterilisiert und/oder sterilisieren kann.

## Beschreibung

Die Erfindung betrifft eine Raumluftheizeinrichtung sowie eine Anordnung einer Raumluftheizeinrichtung.

Raumluftheizeinrichtungen sind bereits aus dem Stand der Technik bekannt. Diese dienen unter anderem dazu, ein in einem Raum befindliches Fluid, insbesondere Luft, aufzuheizen. Problemtisch ist bei den aus dem Stand der Technik bekannten Raumluftheizeinrichtungen, dass durch den heizenden Wärmestrom die Bakterien und die Virenbelastung des Raumes gesteigert wird. Dies führt häufig zu kranken Bewohnern des zu heizenden Raumes.

Es ist daher Aufgabe der vorliegenden Erfindung, eine Raumluftheizeinrichtung bereitzustellen, welche die Gefährdung der Gesundheit von Menschen und Tieren reduzieren soll.

Diese Aufgabe wird mit einer Raumluftheizeinrichtung gemäß dem Anspruch 1 und durch eine Anordnung gemäß dem Anspruch 10 gelöst.

Erfindungsgemäß ist eine Raumluftheizeinrichtung, umfassend eine Luftführungsanordnung und eine Sterilisatoreinheit, wobei die Luftführungsanordnung einen Einlass und einen Auslass aufweist, wobei die Luftführungsanordnung dazu ausgelegt ist, einen Fluidstrom, insbesondere einen Luftstrom, von dem Einlass zu dem Auslass zu leiten, wobei die Luftführungsanordnung zumindest eine beheizbare und/oder beheizte Wand aufweist, wobei die Sterilisatoreinheit derart angeordnet ist, dass diese den Fluidstrom sterilisiert und/oder sterilisieren kann. Die Erfindungsgemäße Raumluftheizeinrichtung dient dabei insbesondere dazu, eine Raumluft, beispielsweise eines Raums eines Gebäudes, aufzuheizen bzw. zu beheizen. Die Raumluftheizeinrichtung umfasst zumindest eine Luftführungsanordnung, welche wiederrum einen Einlass und einen Auslass aufweist, wobei ein Fluidstrom von dem Einlass zu dem Auslass durch die Luftführungsanordnung geleitet werden kann. Der Einlass dient daher insbesondere als ein Eintritt und der Auslass als ein Austritt eines Fluidstroms von bzw. aus der Umgebung in die Luftführungsanordnung. Vorteilhafterweise erstreckt sich die Luftführungsanordnung zumindest teilweise durch einen Bereich der Raumluftheizeinrichtung. Alternativ oder zusätzlich bevorzugt kann die Luftführungsanordnung auch an einer äußerlich zugänglichen Stelle angeordnet sein, welche insbesondere zusammen mit einer bauseitigen Wand, beispielsweise des zu heizenden Raums, in einer endgültigen Anordnungsstellung bzw. in einem montierten Zustand einen Kanal ausbildet. In anderen Worten kann die Luftführungsanordnung einen Kanal durch die Raumluftheizeinrichtung ausbilden und/oder zusammen mit einer bauseitigen Wand einen Kanal ausbilden. Die Luftführungsanordnung dient insbesondere dazu, einen Fluidstrom von dem Einlass zu dem Auslass der Luftführungsanordnung zu leiten. In anderen Worten kann die Luftführungsanordnung daher dazu dienen, die Richtung bzw. den Strömungsweg des Fluidstroms zu begrenzen und/oder zumindest teilweise vorzugeben und/oder den Fluidstrom zwischen dem Einlass und dem Auslass zu leiten. Der Fluidstrom ist insbesondere ein Luftstrom, welcher von dem zu heizenden Raum in den Einlass über die Luftführungsanordnung zum Auslass in den oder die zu beheizenden Räume geführt wird. Um den vom Einlass zum Auslass der Luftführungsanordnung strömenden Fluidstrom zu beheizen, verfügt die Luftführungsanordnung über eine beheizbare Wand. Diese Wand kann dabei insbesondere durch eine Heizeinrichtung der Raumluftheizeinrichtung beheizt sein oder beheizbar sein. Eine derartige Heizeinrichtung der Raumluftheizeinrichtung kann beispielsweise ein Radiator, eine Brennkammer, insbesondere eine Gas oder Feststoffbrennkammer, eine Brennkammer für Biomasse oder eine Elektroheizung sein und/oder umfassen. Zweckmäßigerweise weist die Heizeinrichtung der Raumluftheizeinrichtung eine Heizleistung von mindestens 800 Watt, bevorzugt von zumindest 1200 Watt und besonders bevorzugt von mindestens 2000 Watt und besonders stark bevorzugt von mindestens 4000 Watt auf. Vorteilhafterweise ist die beheizbare Wand dabei direkt oder indirekt durch die Heizeinrichtung der Raumluftheizeinrichtung beheizbar. In anderen Worten kann daher die beheizbare Wand derart ausgebildet sein, dass diese durch die Heizeinrichtung der Raumluftheizeinrichtung beheizbar ist. Die Raumluftheizeinrichtung verfügt auch über eine Sterilisatoreinheit, welche derart angeordnet ist, dass diese den Fluidstrom sterilisiert und/oder sterilisieren kann. Unter einem Sterilisieren kann dabei in diesem Zusammenhang verstanden werden, dass die Bakterienbelastung und/oder die Virenlast des Fluidstroms durch die Sterilisatoreinheit reduziert wird. Eine derartige Sterilisatoreinheit kann beispielsweise eine UV-Lampe, ein, insbesondere sehr engmaschiges, Reinigungsgitter, eine Desinfektionsmittelsprüheinheit und/oder eine zusätzliche Heizeinrichtung sein und/oder umfassen. Durch die Anordnung der Sterilisatoreinheit derart, dass der vom Einlass zum Auslass strömende beheizte bzw. beheizbare Fluidstrom durch die Sterilisatoreinheit sterilisiert wird und/oder sterilisierbar ist, kann erreicht werden, dass die Viren- und/oder Bakterienbelastung durch die Beheizung reduziert werden kann. Daher kann durch die erfindungsgemäße Raumluftheizeinrichtung eine deutliche Reduktion der Gefährdung der Gesundheit von Menschen und Tieren in dem zu beheizenden bzw. dem beheizten Raum erreicht werden.

Vorzugsweise ist der Fluidstrom, insbesondere ausschließlich, durch Konvektion getrieben und oder treibbar. In anderen Worten kann die Raumluftheizeinrichtung derart ausgebildet sein, dass durch diese der zu heizende Fluidstrom, welcher vorteilhafterweise zumindest teilweise durch die Raumluftheizeinrichtung fließen kann, zumindest teilweise, bevorzugt vollständig, durch die Aufwärmung des Fluidstroms in der Luftführungsanordnung vom Einlass zum Auslass strömt. Daher kann die Raumluftheizeinrichtung insbesondere derart ausgebildet sein, dass der Luftstrom bzw. der zu beheizende Fluidstrom, vorteilhafterweise ausschließlich, durch die Beheizung der beheizbaren Wand von dem Einlass zum Auslass getrieben ist. Alternativ oder zusätzlich bevorzugt kann die Raumluftheizeinrichtung oder zumindest die Luftführungsanordnung, insbesondere zwischen Einlass und Auslass, lüfterlos ausgebildet sein. Durch die Ausnutzung von (natürlicher) Konvektion zum Antreiben des Fluidstroms kann eine besonders leise Strömungsantreibung des Fluidstroms, insbesondere des zu beheizenden Luftstroms, zwischen dem Einlass und dem Auslass erreicht werden. Um die (natürliche) Konvektion als Strömungstreiber besonders effektiv ausnutzen zu können, ist es bevorzugt, wenn der Einlass unterhalb des Auslasses angeordnet ist. In anderen Worten kann dies bedeuten, dass der Einlass und der Auslass relativ zueinander derart angeordnet sind, dass in einem montierten Zustand bzw. einem angeordneten Zustand, in welchem die Raumluftheizeinrichtung insbesondere in der Lage ist, den Fluidstrom zwischen dem Einlass und dem Auslass zu beheizen, der Einlass in Richtung entgegen des Vektors der Erdbeschleunigung vom Auslass beabstandet ist. Daher kann insbesondere die maximale Höhe des Einlasses kleiner als die minimale Höhe des Auslasses - in einem montieren Zustand - sein.

Alternativ oder zusätzlich bevorzugt kann die Raumluftheizeinrichtung, vorzugsweise innerhalb der Luftführungsanordnung, eine Fördereinrichtung aufweisen, wobei der Fluidstrom von dem Einlass zu dem Auslass durch die Fördereinrichtung gefördert ist oder förderbar ist. In anderen Worten kann die Raumluftheizeinrichtung daher eine Einrichtung aufweisen, welche den Fluidstrom vom Einlass zum Auslass (zwangsweise) fördert. Beispielsweise kann daher die Fördereinrichtung einen Ventilator und/oder Schaufeln (Blades) aufweisen. Durch die Verwendung einer Fördereinrichtung kann in besonders einfacher Weise erreicht werden, dass ein Fluidstrom vom Einlass zum Auslass etabliert wird. Durch eine Kombination des (natürlich) konvektiv getriebenen Fluidstroms und des (zwangsweisen) Antreibens des Fluidstroms durch eine Fördereinrichtung kann erreicht werden, dass die Fördereinrichtung besonders gering dimensioniert werden kann, sodass der Energiebedarf und/oder die Geräuschbelästigung der Fördereinrichtung reduziert werden kann.

Vorteilhafterweise weist die Raumluftheizeinrichtung eine Brennkammer auf. In anderen Worten kann die Heizeinrichtung der Raumluftheizeinrichtung einen Raum zum Verbrennen von Materialien aufweisen. Zweckmäßigerweise ist diese Brennkammer eine Feststoffbrennkammer, um hohe Energieleistungen bereitstellen zu können. In anderen Worten kann die Brennkammer daher insbesondere derart beschaffen sein, dass diese kein Gas verbrennt oder andere flüchtige, insbesondere fluidische, Brennstoffe. Beispielsweise kann die Brennkammer daher eine Brennkammer zur Verbrennung von Kohle oder Holz darstellen. Alternativ oder zusätzlich bevorzugt kann die Brennkammer eine Biomasseverwertungsbrennkammer darstellen, sodass in der Brennkammer beispielsweise Holzpellets und/oder Holzscheite verbrennbar sind. Hierdurch kann insbesondere eine CO2-günstige Heizbarkeit erreicht werden.

In einer bevorzugten Ausführungsform ist die Brennkammer eine raumluftunabhängige Kammer. In anderen Worten kann dies bedeuten, dass die Raumluftheizeinrichtung derart ausgebildet ist, dass diese für die Bereitstellung von Sauerstoff für die Verbrennung in der Brennkammer keine Verbindung mit der umgebenden Raumluft verwendet und/oder aufweist. Durch die Ausbildung der Raumluftheizeinrichtung derart, dass die Brennkammer eine raumluftunabhängige Brennkammer ist, kann der Verbrauch von Raumluft für die Verbrennung vermieden werden, sodass auf häufiges Lüften verzichtet werden kann. Hierdurch kann insbesondere der Energieverlust durch das Lüften reduziert werden, sodass eine besonders klimafreundliche Raumluftheizeinrichtung resultiert. Aufgrund der Sterilisatoreinheit der Raumluftheizeinrichtung kann jedoch trotz eines (möglichen) fehlenden Lüftens dennoch eine Gesundheitsgefährdung reduziert und/oder sogar verhindert werden.

In einer bevorzugten Ausführungsform kann die Brennkammer eine Brennkammer für Biomasse und/oder Festbrennstoffe sein. Durch die Ausbildung der Brennkammer der Raumluftheizeinrichtung dahingehend, dass diese Feststoffe verwerten kann, kann eine besonders kostengünstige und visuell attraktive Brennkammer erreicht werden. Durch die Ausbildung der Brennkammer als eine Brennkammer für Biomasse bzw. eine Biomassenverwertungsbrennkammer kann die Brennkammer als CO2 neutrale Brennkammer betrieben werden und/oder die auftretende CO2 Belastung durch die Verwendung nachwachsender Rohstoffe reduziert werden. Die Biomasse kann dabei beispielsweise Brennholz und/oder Holzpellets sein.

Vorteilhafterweise ist zumindest eine der beheizbaren und/oder der beheizten Wände, zumindest teilweise, durch eine Abgasleitung, insbesondere eine Abgasleitung der Brennkammer, ausgebildet. In anderen Worten kann zumindest eine der beheizbaren Wände der Luftführungsanordnung derart ausgebildet sein, dass diese zumindest teilweise einen Abgasstrom, insbesondere von der Brennkammer der Heizeinrichtung der Raumluftheizeinrichtung, ausgebildet. Beispielsweise kann zumindest eine der beheizbaren Wände der Luftführungsanordnung derart ausgebildet sein, dass diese zumindest teilweise einen Abgasstrom und/oder eine Abgasleitung, insbesondere von der Brennkammer der Raumluftheizeinrichtung, umgibt. Hierdurch kann in effektiver Weise die in dem Abgas enthaltene Wärmeenergie effizient genutzt werden. Alternativ bevorzugt oder zusätzlich bevorzugt kann auch zumindest eine der beheizbaren und/oder der beheizten Wände eine Wand einer Feuerstätte sein, insbesondere eine Wand einer Brennkammer einer derartigen Feuerstätte.

Zweckmäßigerweise ist die zumindest eine, bevorzugt alle, der beheizbaren und/oder der beheizten Wände durch die Brennkammer und/oder durch die Heizeinrichtung der Raumluftheizeinrichtung beheizt oder beheizbar. In anderen Worten kann dies bedeuten, dass die Brennkammer bzw. die Heizeinrichtung der Raumluftheizeinrichtung die beheizbaren Wände oder die zumindest eine beheizbare Wand beheizen. Daher sind insbesondere keine weiteren und/oder externe Heizeinrichtungen vorgesehen, welche dazu dienen, die Wände der Luftführungsanordnung zu beheizen. Unter der Begrifflichkeit Heizeinrichtung im Sinne der Erfindung sollen jedoch kleinere Wärmequellen, wie beispielsweise Glühbirnen oder UV-Lampen, nicht verstanden sein. Kleinere Wärmequellen weisen insbesondere ein Heizleistung von weniger als 200 Watt, bevorzugt von weniger als 100 Watt, und besonders bevorzugt von weniger als 50 Watt, auf.

Vorteilhafterweise bildet zumindest eine der beheizbaren und/oder der beheizten Wände, zumindest teilweise, eine Verkleidungswand, insbesondere eine Verkleidung der Brennkammer, aus. In anderen Worten kann die beheizbare Wand selbst ein Teil der Feuerstätte bzw. der Brennkammer, insbesondere einer Außenwand der Brennkammer, ausbilden und/oder die Brennkammer gegenüber der Luftführungsanordnung verkleiden. Hierdurch kann eine besonders effektive und direkte Beheizbarkeit der zu beheizenden Wand durch die Brennkammer erreicht werden, sodass eine besonders energieeffiziente Wärmeübertragung bei hohen Temperaturen stattfinden kann.

Vorteilhafterweise ist zumindest eine der beheizbaren und/oder der beheizten Wände aus Metall ausgebildet. Hierdurch kann eine besonders effektive Wärmeleitung erreicht werden.

Alternativ oder zusätzlich bevorzugt kann zumindest eine der beheizbaren und/oder der beheizten Wände aus Beton ausgebildet sein. Hierdurch kann eine besonders kostengünstige beheizbare bzw. beheizte Wand erreicht werden.

Vorteilhafterweise weist die Raumluftheizeinrichtung eine Sichtausnehmung oder ein Sichtfenster auf, wobei die Sichtausnehmung oder das Sichtfenster insbesondere eine Einsicht in den Brennraum der Brennkammer bzw. in die Brennkammer von der Umgebung aus ermöglicht. Hierdurch kann eine besonders einfache Inspektion des Zustandes der Raumluftheizeinrichtung erreicht werden. Vorteilhafterweise weist die Sichtausnehmung und/oder das Sichtfenster eine Blickfläche von mindestens 300 cm², bevorzugt von mindestens 600 cm², und besonders bevorzugt von mindestens 800 cm², auf. Die Blickfläche ist insbesondere die Fläche der blickdurchlässigen Fläche des Sichtfensters bzw. der Sichtausnehmung. Bei einer Blickfläche von mindestens 300 cm² kann eine besonders einfache Inspektion der Brennkammer erfolgen. Sollte die Blickfläche mindestens 600 cm² betragen, so kann hierdurch eine Erhellung der Umgebung stattfinden, sodass Beleuchtungsenergie gespart werden kann. Sollte die Blickfläche mindestens 800 cm² betragen, so kann hierdurch ein besonders behagliches Raumgefühl bei einem Betrachter erreicht werden.

Vorteilhafterweise weise die Raumluftheizeinrichtung einen Korpus, insbesondere einen Betonkorpus, auf. Der Korpus begrenzt insbesondere die Raumluftheizeinrichtung gegenüber der Umgebung. Vorteilhafterweise weist der Korpus eine Wandstärke auf, welche insbesondere mindestens 1 cm, bevorzugt zumindest 1,5 cm und besonders stark bevorzugt zumindest 2 cm beträgt. Bei einer Wandstärke von mindestens 1 cm kann eine besonders gute Isolationswirkung erreicht werden, sodass Verbrennungen verhindert werden können oder zumindest deren Gefahr reduziert werden kann. Bei einer Wandstärke von mindestens 1.5 cm kann eine besonders gute Festigkeit des Korpus erreicht werden. Sollte die Wandstärke mindestens 2 cm betragen, so kann hierdurch eine besonders hohe Standsicherheit erreicht werden. Maßgeblich für die Wandstärke ist dabei insbesondere die maximale, die minimal oder die gemittelte Wandstärke des Korpus, wobei diesbezüglich insbesondere die Wände des Korpus entscheidend sein können, welche den Korpus gegenüber der Umgebung abgrenzen.

Zweckmäßigerweise weißt die Raumluftheizeinrichtung einen Betonkorpus auf. In anderen Worten kann die Raumluftheizeinrichtung derart ausgebildet sein, dass insbesondere die äußeren umgebenden Wände der Raumluftheizeinrichtung zum überwiegenden, bevorzugt zumindest zu 60 Prozent und besonders bevorzugt zu zumindest 70 Prozent, aus Beton ausgebildet sind. Hierdurch kann eine besonders kostengünstige Ausbildung der Raumluftheizeinrichtung erreicht werden. Zweckmäßigerweise verlaufen dabei Bewehrungen durch den Betonkorpus, insbesondere in Richtung der Längsrichtung des Korpus bzw. der Raumluftheizeinrichtung. Die Längsrichtung ist dabei insbesondere diejenige Richtung, in welche sich die Länge des Korpus und/oder die Länge der Raumluftheizeinrichtung bemisst. Alternativ oder zusätzlich bevorzugt kann die Längsrichtung auch diejenige Richtung sein, in welche die Raumluftheizeinrichtung bzw. der Korpus der Raumluftheizeinrichtung seine größte Hauptdimension aufweist.

Zweckmäßigerweise ist der Einlass der Luftführungsanordnung unterhalb des Auslasses angeordnet. Hierdurch kann, wie bereits beschrieben, eine besonders vorteilhafte Ausbildung der Luftführungsanordnung erreicht werden, denn bei einer derartigen Ausbildung kann die natürliche Konvektion durch das Aufheizen des Fluidstroms genutzt werden, um den Fluidstrom konvektiv anzutreiben. Besonders bevorzugt ist es, wenn der Einlass und der Auslass an bzw. in der gleichen Wand der Raumluftheizeinrichtung, insbesondere der gleichen Wand des Korpus der Raumluftheizeinrichtung, angeordnet sind. Hierdurch kann in besonders bauraumsparender Weise erreicht werden, dass der Einlass und der Auslass der Raumluftheizeinrichtung in einem montierten Zustand in denselben zu heizenden Raum münden.

Zweckmäßigerweise weist die Sterilisatoreinheit einen Energieanschluss, insbesondere einen Stromanschluss, auf. In anderen Worten kann die Sterilisatoreinheit als ein Energieverbraucher ausgebildet sein. Hierdurch kann in besonders effektiver Weise eine gezielte, insbesondere eine schaltbare, Sterilisatoreinheit ausgebildet werden, welche insbesondere mit keinen (körperlichen) Verbrauchsgütern, wie beispielsweise Desinfektionsmittel, versorgt werden muss. Daher kann durch eine derartige Ausbildung eine besonders kompakte und einfach zu betreibende Sterilisatoreinheit erreicht werden.

Zweckmäßigerweise ist die Sterilisatoreinheit eine UV-Lampe, insbesondere eine UV-C-Lampe, und/oder umfasst eine UV-Lampe, insbesondere eine UV-C-Lampe. Durch die Verwendung einer UV-Lampe, insbesondere einer UV-C-Lampe, kann eine besonders effektive und einfache Reduktion der Viren- und/oder der Bakterienbelastung des Fluidstroms in der Luftführungsanordnung erreicht werden, welche insbesondere keine oder nur eine geringe Umweltbelastung verursacht. Besonders effektiv ist dabei, dass durch die Raumluftheizeinrichtung der Fluidstrom aufgewärmt wird, denn überraschender Weise hat sich herausgestellt, dass eine Sterilisation durch UV-Strahlung bei hohen Temperaturen besonders effektiv und/oder zumindest begünstigt sein kann. Zweckmäßigerweise ist daher, um eine besonders hohe Temperatur im Sterilisationsbereich zu erreichen, die UV-Lampe innerhalb der Luftführungsanordnung, insbesondere in einem Kanal der Luftführungsanordnung, angeordnet.

In einer vorteilhaften Ausbildung ist die Sterilisatoreinheit derart ausgebildet und/oder angeordnet, dass zumindest 30 Prozent, bevorzugt zumindest 70 Prozent und besonders bevorzugt zumindest 80 Prozent des Fluidstroms sterilisiert und/oder sterilisierbar durch die Sterilisatoreinheit sind. In anderen Worten kann dies bedeuten, dass die Sterilisatoreinheit derart angeordnet und/oder ausgebildet ist, dass diese einen bedeutenden Teil des Massen- und/oder des Volumenstroms des zu fördernden Fluidstroms sterilisieren kann. Maßgeblich für die Prozentverhältnisse sind dabei insbesondere die prozentualen Volumen- und/oder Massenverhältnisse des austretenden Fluidstroms aus dem Auslass bzw. den Auslässen der Raumluftheizeinrichtung. Alternativ bevorzugt oder zusätzlich bevorzugt kann für die prozentualen Verhältnisse auch die Querschnittsfläche desjenigen Querschnitts der Luftführungsanordnung maßgeblich sein, welcher durch die Sterilisatoreinheit sterilisiert wird.

Zweckmäßigerweise ist die Sterilisatoreinheit blickdicht, insbesondere an einem blickdichten Bereich der Luftführungsanordnung, angeordnet. Unter einer blickdichten Anordnung ist dabei zu verstehen, dass in einem montierten bzw. angeordneten Zustand der Raumluftheizeinrichtung eine direkte visuelle Betrachtung der Sterilisatoreinheit nicht möglich ist. In anderen Worten kann dies bedeuten, dass bei einer blickdichten Anordnung der Sterilisatoreinheit ein direktes Hineinblicken in die Sterilisatoreinheit, welche z.B. eine UV-C-Lampe sein kann, durch die Sterilisatoreinheit und/oder durch andere Bestandteile der Raumluftheizeinrichtung verhindert ist. Beispielsweise kann die Sterilisatoreinheit und/oder die Raumluftheizeinrichtung einen Sichtschutz aufweisen, um eine blickdichte Anordnung zu erreichen.

Bevorzugter Weise bildet die Luftführungsanordnung zumindest abschnittsweise, bevorzugt vollständig, einen Kanal zwischen dem Einlass und dem Auslass aus, wobei der Kanal zumindest teilweise durch zumindest eine beheizbare und/oder beheizte Wand ausgebildet ist. Unter einem Kanal ist dabei zu verstehen, dass der zwischen dem Einlass und dem Auslass strömende Fluidstrom durch die Wände des Kanals vollständig umgeben ist. Ein Kanal ist daher insbesondere dann gegeben, wenn in einem Querschnitt der durch den Kanal strömende Fluidstrom vollständig durch Wände umgeben ist. In anderen Worten kann die Luftführungsanordnung derart ausgebildet sein, dass diese durch Wände, welche den Kanal ausbilden, vollständig umgeben ist und nur Einlässe und Auslässe eine fluidische Verbindung zu einer Umgebung bereitstellen. Zweckmäßigerweise ist zumindest eine der Wände des Kanals eine beheizbare bzw. beheizte Wand. Hierdurch kann eine besonders effektive Einbringung eines Wärmestroms in den Fluidstrom erreicht werden, sodass eine besonders kompakte Luftführungsanordnung bzw. Raumluftheizeinrichtung resultiert.

Zweckmäßigerweise ist die Raumluftheizeinrichtung derart ausgebildet, dass eine visuelle Inspektion des Inneren der Brennkammer, beispielsweise durch ein Sichtfenster, möglich ist. In anderen Worten kann die Heizeinrichtung bzw. die Brennkammer ein Sichtfenster aufweisen. Vorteilhafterweise ist das Sichtfenster dabei in einer Brennkammertür bzw. einer Heizeinrichtungstür angeordnet. Hierdurch kann eine besonders einfache Nachfüllung und eine einfache visuelle Inspektion der Brennkammer erreicht werden.

Ein weiterer Aspekt der Erfindung kann eine Anordnung einer Raumluftheizeinrichtung, insbesondere wie vorgehend und nachfolgend beschrieben, in einem Raum betreffen. Dieser Raum umschließt vorteilhafterweise einen Volumen, wobei der Einlass und/oder der Auslass der Luftführungsanordnung der Raumluftheizeinrichtung direkt die Luftführungsanordnung mit dem Volumen des Raums verbindet bzw. verbinden. In anderen Worten kann die in dem Raum enthaltene Luft direkt durch den Einlass in die Luftführungsanordnung der Raumluftheizeinrichtung und/oder aus dem Auslass in das Volumen des Raums strömen, ohne durch weitere Strömungsleitungen oder Führungsanordnungen geführt werden zu müssen.

Hierdurch kann eine besonders kompakte Beheizbarkeit des Volumens des Raumes erreicht werden. Der Raum ist dabei insbesondere der Raum eines Gebäudes, insbesondere eines Wohngebäudes.

Weitere Vorteile und Merkmale der vorliegenden Erfindung ergeben sich aus der nachfolgenden Beschreibung mit Bezug auf die Figuren. Einzelne Merkmale der dargestellten Ausführungsformen können dabei auch in anderen Ausführungsformen eingesetzt werden, sofern dies nicht ausdrücklich ausgeschlossen wurde. Es zeigen:
- Figur 1: eine isometrische Ansicht einer Raumluftheizeinrichtung, mit einem Detailausbruch;
- Figur 2: eine Detailansicht einer Raumluftheizeinrichtung im Bereich der Sterilisatoreinheit;
- Figur 3: eine weitere perspektivische Ansicht einer Raumluftheizeinrichtung, ohne Detailausbruch;
- Figur 4: eine schematische Schnittansicht durch eine Raumluftheizeinrichtung;
- Figur 5: eine weitere schematische Schnittansicht durch eine Raumluftheizeinrichtung; und
- Figur 6: eine zusätzliche Schnittansicht durch eine beispielhafte Raumluftheizeinrichtung.

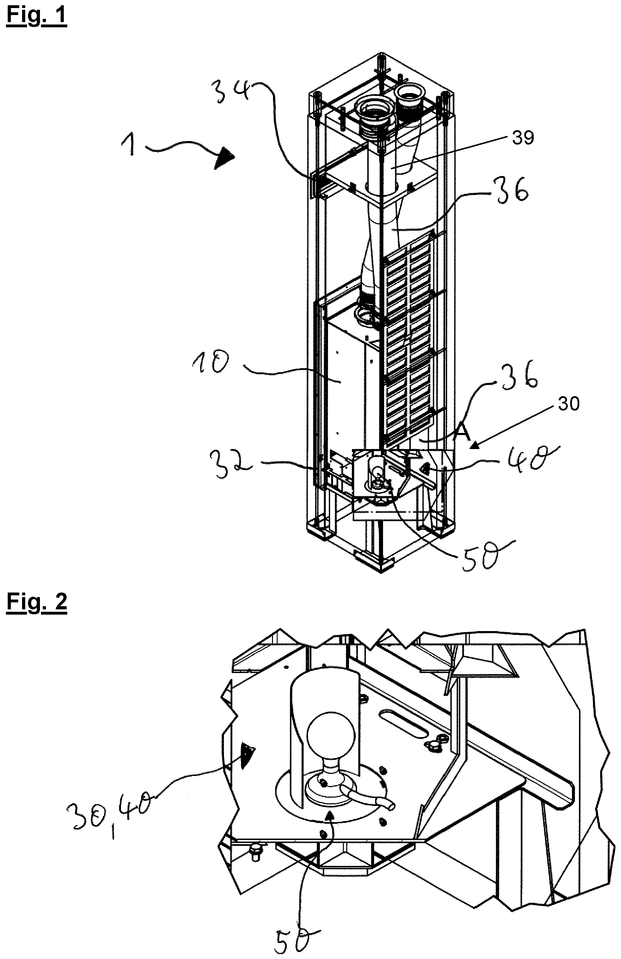

In der **Figur 1** ist eine Raumluftheizeinrichtung 1 gezeigt, welche eine Heizeinrichtung mit einer Brennkammer 10 aufweist. Diese Brennkammer 10 ist als eine raumluftunabhängige Brennkammer 10 ausgebildet. Daher ist die Brennkammer 10 über eine Abgasleitung 39 und eine Frischluftzufuhr mit einem ein Gebäude umgebenden Luftbereich verbunden. Die Raumluftheizeinrichtung 1 verfügt über eine Luftführungsanordnung 30, welcher sich vollständig innerhalb der Raumluftheizeinrichtung 1 erstreckt. Die Luftführungsanordnung 30 verfügt über einen Einlass 32 und einen Auslass 34, wobei der Einlass 32 vollständig unterhalb des Auslasses 34 angeordnet ist. Innerhalb der Luftführungsanordnung 30 der Raumluftheizeinrichtung 1 sind zahlreiche beheizbare Wände 36 ausgebildet. Eine der beheizbaren Wände 36 ist eine Außenwand der Brennkammer 10 und eine weitere beheizbare Wand 36 der Luftführungsanordnung 30 ist durch die Abgasleitung der Brennkammer 10 ausgebildet. Die Luftführungsanordnung 30 verfügt über zumindest einen Kanal 40, welcher sich unterhalb der Brennkammer 10 erstreckt. Innerhalb dieses Kanals 40 ist eine Sterilisatoreinheit 50 angeordnet. Die Sterilisatoreinheit 50 verfügt über einen Blickschutz sowie über eine UV-C Lampe.

In der **Figur 2** ist eine Detailansicht des in der Figur 1 mittels eines viereckigen Kastens ausgeschnittenen Bereichs (A) dargestellt, um eine detailliertere Ansicht der Sterilisatoreinheit 50 zu ermöglichen. Wie der Figur 2 zu entnehmen ist, befindet sich die Sterilisatoreinheit 50 innerhalb des Kanals 40 der Luftführungsanordnung 30. Um eine blickdichte Anordnung der Sterilisatoreinheit 50 in der Luftführungsanordnung 30 zu erreichen, ist ein Sichtschutz vorgesehen, welcher einen direkten Blick in die UV-C Lampe der Sterilisatoreinheit 50 verhindert. In dem dargestellten Ausführungsbeispiel in der Figur 2 ist die Sterilisatoreinheit 50 derart ausgebildet, dass diese zumindest 80 Prozent des Fluidstroms durch die Luftführungsanordnung 30 sterilisiert und/oder sterilisieren kann, denn zumindest 80 Prozent des Strömungsquerschnitts des Kanals 40 sind durch die von der UV-C Lampe emittieren Strahlen behandelt bzw. sterilisiert.

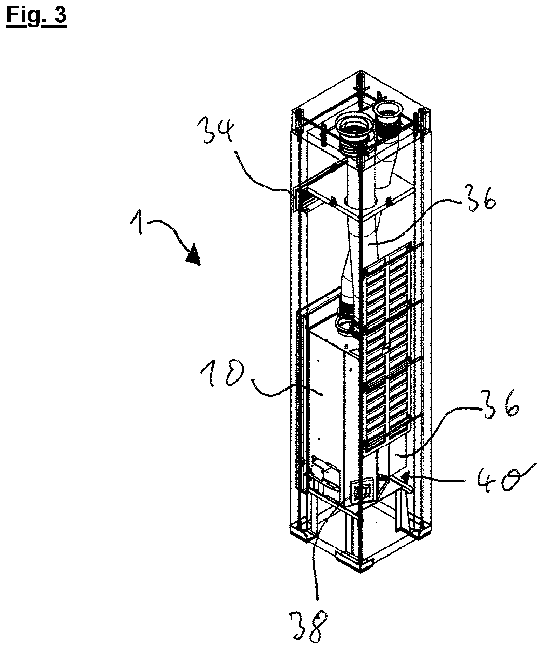

In der **Figur 3** ist eine weitere Ausführungsvariante einer Raumluftheizeinrichtung 1 gezeigt, wobei die in der Figur 3 dargestellte Ausführungsform prinzipiell zu den in der Figur 1 und/oder der Figur 2 dargestellten Ausführungsvarianten passen kann. In der Figur 3 ist jedoch kein Ausbruch dargestellt, welcher eine Sicht auf die Sterilisatoreinheit 50 ermöglicht. Die in der Figur 3 dargestellte Raumluftheizeinrichtung 1 verfügt über eine Fördereinrichtung 38, welche zumindest einen Ventilator aufweist.

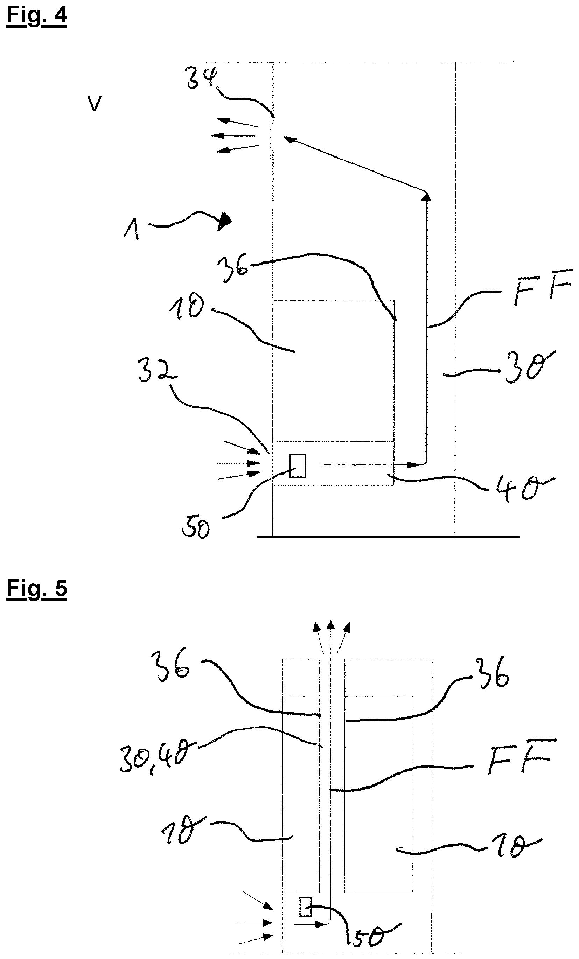

In der **Figur 4** ist ein Schnitt durch eine prinzipielle Skizze einer Raumluftheizeinrichtung 1 gezeigt. Wie der Figur 4 entnehmbar ist, kann ein Fluidstrom FF vom Einlass 32 über den Kanal 40 der Luftführungsanordnung 30 zu dem Auslass 34 strömen. Innerhalb der Raumluftheizeinrichtung 1 ist eine Brennkammer 10 angeordnet, welche eine Rückwand aufweist, wobei diese Rückwand eine beheizbare Wand 36 der Luftführungsanordnung 30 darstellt. Innerhalb des Kanals 40, welcher unterhalb der Brennkammer 10 angeordnet ist, ist eine Sterilisatoreinheit 50 angeordnet, welche in der Lage ist bzw. derart ausgebildet ist, den Fluidstrom FF zu sterilisieren. In der in der Figur 4 dargestellten Ausführungsvariante ist der Fluidstrom FF ausschließlich durch Konvektion getrieben, sodass gerade keine Fördereinrichtung 38 vorhanden ist, welche den Fluidstrom FF (zwangsweise) fördert bzw. etabliert.

In der **Figur 5** ist eine weitere schematische Ansicht einer Raumluftheizeinrichtung 1 dargestellt. In dem dargestellten Ausführungsbeispiel strömt der Fluidstrom FF durch einen Kanal 40, welcher sich durch die Brennkammer 10 der Heizeinrichtung der Raumluftheizeinrichtung 1 erstreckt. Der Kanal 40 weist daher eine Vielzahl von beheizbaren Wänden 36 auf, welche unmittelbar den Kanal 40, sowie den Brennraum der Brennkammer 10 begrenzen. Hierdurch kann eine besonders effektive Wärmestromübertragung in den Fluidstrom FF erreicht werden. Unterhalb der Brennkammer 10 ist eine Sterilisatoreinheit 50 der Raumluftheizeinrichtung 1 angeordnet.

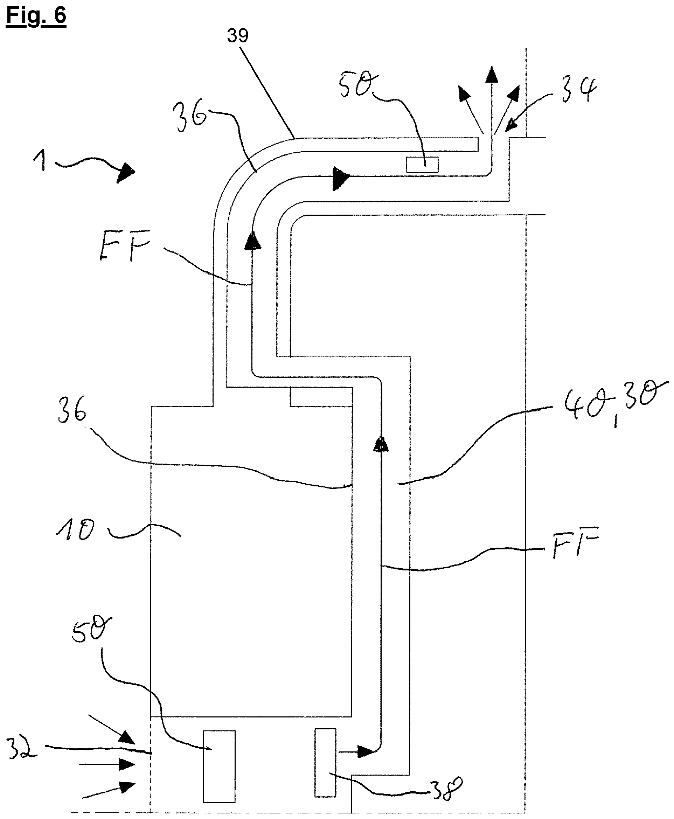

In der **Figur 6** ist eine prinzipielle Anordnung einer Raumluftheizeinrichtung 1 in einem Raum dargestellt, wobei die Darstellung in der Figur 6 dabei eine Schnittansicht ist. Die Raumluftheizeinrichtung 1 verfügt über eine Luftführungsanordnung 30, welche zumindest einen Einlass 32 und zumindest einen Auslass 34 aufweist. Innerhalb der Luftführungsanordnung 30 sind zwei Sterilisatoreinheiten 50 angeordnet, wobei die erste Sterilisatoreinheit 50 in der Nähe des Einlasses 32 und die weitere Sterilisatoreinheit 50 in der Nähe des Auslasses 34 angeordnet sein kann, wie in der Figur 6 prinzipiell dargestellt. Die Luftführungsanordnung 30 ist teilweise von der Abgasleitung 39 umgeben. Die Luftführungsanordnung 30 verfügt über einen Kanal 40, wobei der Kanal 40 zumindest teilweise innerhalb eines Abgasrohres der raumluftunabhängigen Brennkammer 10 angeordnet ist. Innerhalb der Luftführungsanordnung 30 befindet sich eine Fördereinrichtung 38, welche den Fluidstrom FF in einem eingeschalteten Zustand fördert. Die Brennkammer 10 ist insbesondere eine Biomassebrennkammer. In anderen Worten kann die Brennkammer derart ausgebildet sein, dass diese explizit für die Verwertung von Holzbrennstoff, insbesondere Pellets und/oder Holzscheiten, ausgebildet ist. Zweckmäßigerweise ist die Raumluftheizeinrichtung 1 derart ausgebildet, dass eine visuelle Inspektion des Inneren der Brennkammer 10, beispielsweise durch ein Sichtfenster, möglich ist.

### Bezugszeichenliste:

- 1: - Raumluftheizeinrichtung
- 10: - Brennkammer
- 30: - Luftführungsanordnung
- 32: - Einlass
- 34: - Auslass
- 36: - beheizbare Wand
- 38: - Fördereinrichtung
- 39: - Abgasleitung
- 40: - Kanal
- 50: - Sterilisatoreinheit
- FF: - Fluidstrom
- V: - Volumen des Raumes

## Patentansprüche

1. Raumluftheizeinrichtung (1),
umfassend eine Luftführungsanordnung (30) und eine Sterilisatoreinheit (50), wobei die Luftführungsanordnung (30) einen Einlass (32) und einen Auslass (34) aufweist,
wobei die Luftführungsanordnung (30) dazu ausgelegt ist, einen Fluidstrom (FF), insbesondere einen Luftstrom, von dem Einlass (32) zu dem Auslass (34) zu leiten,
wobei die Luftführungsanordnung (30) zumindest eine beheizbare und/oder beheizte Wand (36) aufweist,
wobei die Sterilisatoreinheit (50) derart angeordnet ist, dass diese den Fluidstrom (FF) sterilisiert und/oder sterilisieren kann.

2. Raumluftheizeinrichtung (1) gemäß einem der vorhergehenden Ansprüche, wobei die Raumluftheizeinrichtung (1), vorzugsweise innerhalb der Luftführungsanordnung (30), eine Fördereinrichtung (38) aufweist,
wobei der Fluidstrom (FF) von dem Einlass (32) zu dem Auslass (34) durch die Fördereinrichtung (38) gefördert ist oder förderbar ist.

3. Raumluftheizeinrichtung (1) gemäß einem der vorhergehenden Ansprüche, wobei die Raumluftheizeinrichtung (1) eine Brennkammer (10) aufweist.

4. Raumluftheizeinrichtung (1) gemäß Anspruch 3,
wobei die Brennkammer (10) eine Brennkammer für Biomasse und/oder Festbrennstoffe ist.

5. Raumluftheizeinrichtung (1) gemäß einem der vorhergehenden Ansprüche, wobei die zumindest eine, bevorzugt alle, der beheizbaren und/oder der beheizten Wände (36) durch die Brennkammer (10) beheizt sind oder beheizbar sind.

6. Raumluftheizeinrichtung (1) gemäß einem der vorhergehenden Ansprüche, wobei zumindest eine der beheizbaren und/oder der beheizten Wände (36) aus Beton ist.

7. Raumluftheizeinrichtung (1) gemäß einem der vorhergehenden Ansprüche, wobei die Sterilisatoreinheit (50) einen Energieanschluss, insbesondere einen Stromanschluss, aufweist.

8. Raumluftheizeinrichtung (1) gemäß einem der vorhergehenden Ansprüche, wobei die Sterilisatoreinheit (50) eine UV Lampe ist und/oder umfasst.

9. Raumluftheizeinrichtung (1) gemäß einem der vorhergehenden Ansprüche, wobei die Sterilisatoreinheit (50) blickdicht, insbesondere in einem blickdichten Bereich der Luftführungsanordnung (30), angeordnet und/oder ausgebildet ist.

10. Anordnung einer Raumluftheizeinrichtung (1), insbesondere gemäß einem der vorhergehenden Ansprüche, in einem Raum,
wobei der Raum ein Volumen (V) umschließt,
wobei der Einlass (32) und/oder der Auslass (34) der Luftführungsanordnung (30) direkt die Luftführungsanordnung (30) mit dem Volumen (V) verbindet und/oder verbinden.
